# EUROPÄISCHE PATENTSCHRIFT

(11) **EP 1 126 014 B1**
(45) Veröffentlichungstag und Bekanntmachung des Hinweises auf die Patenterteilung: **28.01.2004**
(21) Anmeldenummer: 01101917.1
(22) Anmeldetag: 27.01.2001
(51) Int. Cl.: C11D 3/48, C11D 3/30, C11D 3/33, C11D 1/835, C11D 1/62

(54) **Reinigungs- und Desinfektionssysteme für medizinische Instrumente**
Cleaning and sanitizing systems for medical devices
Systèmes détergents et désinfectants pour instruments médicaux

(30) Priorität: 17.02.2000 DE 10007324; 16.06.2000 DE 10028998
(43) Veröffentlichungstag der Anmeldung: 22.08.2001
(73) Patentinhaber: Bode Chemie GmbH & Co., 22525 Hamburg (DE)
(72) Erfinder: Fehling, Thomas, 22305 Hamburg (DE); Bloss, Richard, Dr., 25462 Rellingen (DE)
(74) Vertreter: Dinné, Erlend

(56) Entgegenhaltungen:
- WO-A-00/71662
- WO-A-96/10069
- WO-A-98/16605
- WO-A-99/15012
- US-A- 5 403 505

## Beschreibung

Die vorliegende Erfindung betrifft flüssige, bevorzugt aldehydfreie Reinigungs- und Desinfektionssysteme mit einem Gehalt an Tensiden, Korrosionsschutzmitteln und weiteren Zusätzen, die die Reinigung verstärken sowie deren Verwendung zur Reinigung und gleichzeitigen oder nachfolgenden Desinfektion von medizinischen Instrumenten, insbesondere zur Reinigung und gleichzeitigen oder nachfolgenden Desinfektion von flexiblen Endoskopen.

Als Desinfektionsmittel werden Stoffe bezeichnet, die zur Desinfektion, d. h. zur Bekämpfung pathogener Mikroorganismen (z. B. Bakterien, Viren, Sporen, Klein- und Schimmelpilze) geeignet sind und zwar im allgemeinen durch Anwendung an der Oberfläche von Haut, Kleidung, Geräten, Räumen, aber auch von Trinkwasser, Nahrungsmitteln, Saatgut (Beizen) und als Bodendesinfektionsmittel.

Besonders lokal anzuwendende Desinfektionsmittel, z. B. zur Wunddesinfektion, werden auch als Antiseptika bezeichnet.

Desinfektionsmittel werden definiert als Stoffe oder Stoffgemische, die bei der Anwendung auf Gegenständen od. Oberflächen diese in einen Zustand versetzen, daß sie keine Infektion mehr verursachen können. Ihre Wirkung muß bakterizid, fungizid, viruzid und sporizid (Sammelbegriff: mikrobizid) sein. Ein Effekt im Sinne der Bakteriostase ist für Desinfektionsmittel unzureichend. Sie sind daher im allgemeinen pantoxisch, d. h. sie entfalten ihre Wirkung gegen alle lebenden Zellen.

Je nach Verwendungszweck teilt man die Desinfektionsmittel ein in solche zur Wäsche-, Flächen-, Instrumenten-, Haut- und Hände- sowie zur Stuhl- und Sputumdesinfektion.

Unter Desinfektionsreiniger versteht man solche Desinfektionsmittel, die auch als Reinigungs- und gegebenenfalls Pflegemittel fungieren.

Unter Berücksichtigung der vielfältigen Forderungen, die an Desinfektionsmittel gestellt werden, wie z. B. breites Wirkungsspektrum, kurze Einwirkungszeiten, Hautverträglichkeit, geringe Toxizität, Materialverträglichkeit kommen nur einige Wirkstoff-Typen für den Einsatz in Betracht:
1. Die wichtigste Wirkstoff-Gruppe sind die Aldehyde (Formaldehyd, Glyoxal, Glutaraldehyd). Sie besitzen ein breites Wirkungsspektrum einschließlich Virus-Wirksamkeit und sporizider Wirkung bei Formaldehyd und Glutaraldehyd. Sie können zu Sensibilisierung insbesondere der Haut und Atmungsorgane führen. Ihr Einsatz ist daher aus toxikologischen Gründen rückläufig. Desinfektionsmittel auf Aldehydbasis weisen darüber hinaus einen charakteristischen, durchdringenden und unangenehmen Geruch auf.
2. Phenol-Derivate besitzen eine gute bakterizide Wirkung, sind aber nicht sporizid. Gegenüber fast allen anderen Desinfektionsmittelwirkstoffen haben sie den Vorzug, durch Schmutz verhältnismäßig wenig beeinflußt zu werden. Sie eignen sich daher bes. zur Stuhldesinfektion. Typische Vertreter sind 2-Biphenylol und p-Chlor-m-kresol (4-Chlor-3-methylphenol). Diese weisen allerdings etliche Nachteile auf, da sie als toxikologisch und ökologisch bedenklich gelten.
3. Alkohole zeichnen sich durch schnelle Wirksamkeit aus, allerdings erst bei relativ hohen Konzentrationen von ca. 40-80%.
4. Von den Halogenen besitzen Chlor und lod eine gewisse Bedeutung als Desinfektionsmittel. Chlor ist von der Wasseraufbereitung und Schwimmbaddesinfizierung her bekannt und damit seine unangenehmen Eigenschaften wie Geruch und Korrosivität. Trotz der ausgezeichneten Wirkung gegen Bakterien, Pilze, Sporen und Viren haben chlorhaltige Desinfektionsmittel im Humanbereich aus den obengenannten Gründen und wegen der starken Chlor-Zehrung durch organ. Substanzen keine starke Verbreitung gefunden. Dagegen werden Hypochlorite, Chlorkalk- und Chlorisocyanursäuren als technische Desinfektionsmittel noch umfänglich benutzt. Iodtinktur wird im medizinischen Bereich als Antiseptikum verwendet.
5. Desinfektionsmittel auf Basis von aktivem Sauerstoff (z. B. Wasserstoffperoxid, Peroxyessigsäure) haben in letzter Zeit wieder etwas an Bedeutung gewonnen.
6. Die quatemären Ammonium-Verbindungen, Kationentenside (Invertseifen) und Amphotenside gehören zur Klasse der Tenside. Sie zeichnen sich durch recht gute Materialverträglichkeit aus, zeigen ein toxikologisch erheblich besseres Profil, geringere Hautsensibilisierung und sind praktisch geruchlos. Ihr Wirkungsspektrum ist dagegen nur begrenzt, sie haben eine nur geringe Wirksamkeit gegenüber Gramnegativen sowie mykobakterien. Hierher gehören z. B. Benzalkoniumchlorid, Cetrimoniumbromid, Cetylpyridiniumchlorid (Hexadecylpyridiniumchlorid), Didecyldimethylammoniumchlorid und andere.
   Quatemäre Ammoniumverbindungen sind organisch Ammoniumverbindungen mit quatemären Stickstoffatomen. Quatemäre Ammoniumverbindungen mit einem hydrophoben Alkyl-Rest sind biozid.
   Quatemäre Ammoniumverbindungen werden durch Umsetzung tertiärer Amine mit Alkylierungsmitteln, wie z. B. Methylchlorid, Benzylchlorid, Dimethylsulfat, Dodecylbromid, aber auch Ethylenoxid hergestellt. In Abhängigkeit von dem eingesetzten tertiären Amin unterscheidet man drei Gruppen: 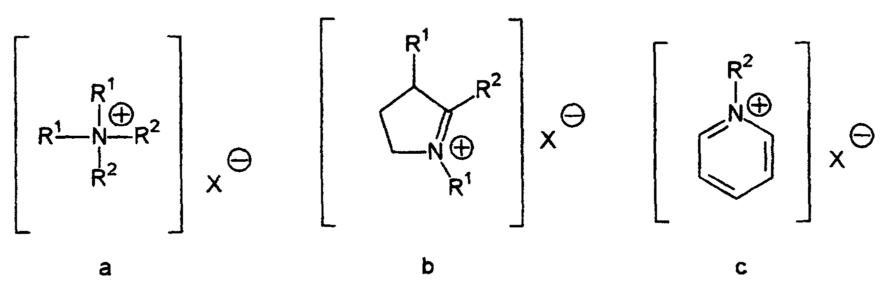
   a) Lineare Alkylammoniumverbindungen
   b) Imidazoliniumverbindungen
   c) Pyridinium-Verbindungen
   Typische Vertreter weisen folgende Substitution auf: R¹ = CH₃, R² = C₈₋₁₈, X = Halogen.
   Die Alkylierung tertiärer Amine mit einem langen Alkylrest und zwei Methylgruppen gelingt besonders leicht, auch die Quatemierung tertiärer Amine mit zwei langen Resten und einer Methylgruppe kann mit Hilfe von Methylchlorid unter milden Bedingungen durchgeführt werden. Amine, die über drei lange Alkylreste oder hydroxysubstituierte Alkylreste verfügen, sind wenig reaktiv und werden bevorzugt mit Dimethylsulfat quaterniert.
7. Weitere Desinfektionsmittel beruhen auf der Verwendung von Alkylaminen. Zu den wichtigsten Vertretern dieser Gruppe zählen Fettaminderivate wie z.B. Lauryl-propylendiamin ("Cocospropylendiamin") und Dodecylbispropylentriamin. Alkylamine zeigen die gleichen Vorteile gegenüber Aldehyden wie quatemäre Ammoniumverbindungen, haben ein hervorragendes Reinigungsvermögen und besitzen ein im Vergleich zu den quaternären Ammoniumverbindungen besseres Wirkungsspektrum gegenüber gramnegativen Bakterien. Sie können aber bei bestimmten Kunststoffmaterialien zu Schädigungen führen und waren daher bisher nicht im kompletten Instrumentenmaterial einsetzbar.

Außer den genannten Mikrobizid-Wirkstoffen sind noch eine Anzahl von mikrobistat. Substanzen und Konservierungsmitteln (Diphenylether, Carbanilide, Acetanilide aromatischen Säuren und deren Salze) für spezifische Verwendung auf dem Markt, die im erweiterten Sinne den Desinfektionsmitteln zugerechnet werden.

Eine einheitliche Wirkungsweise der Desinfektionsmittel ist nicht zu erkennen. Während manche Präparate auf die Cytoplasmamembran der Bakterien zerstörend wirken sollen, wird von anderen eine irreversible Blockierung wichtiger Sulfidbindungen bei Enzymen oder von Spurenelementen (durch Chelatisierung) angenommen.

Endoskope sind röhren- oder schlauchförmige Instrumente, welche zur Untersuchung von Körperhohlräumen und Hohlorganen verwendet werden. Sie sind mit einem optischen System, bestehend aus Objektiv und Okular, einer Beleuchtungseinrichtung und meist Spül- und Absaugvorrichtungen sowie Kanälen zum Einführen spezieller Instrumente ausgestattet. Flexible Endoskope (welche auch als Fiberendoskope bezeichnet werden) sind mit einem Glasfaserlichtleiter ausgerüstet, welcher aus einem dicht gepackten Bündel aus haarfeinen und deshalb hochflexiblen Einzelfasem besteht. Durch die Flexibilität der Glasfasern können Licht und Bild über praktisch jede beliebige Abwinklung übertragen werden. Zusätzlich zu den Faserbündeln und den Bowdenzügen für die Bewegung der Spitze sind Kanäle für Wasserspülung und Luft sowie ein in der Regel größerer Biopsie- und gleichzeitig Absaugkanal untergebracht.

Die Endoskopie kann allein, beispielsweise zur Entnahme von Gewebeproben, aber auch in Kombination mit weiteren Methoden, wie z. B. der Ultraschalldiagnostik (Endosonographie) oder der Röntgendiagnostik durchgeführt werden. Gebräuchlich sind beispielsweise Röntgenkontrastdarstellungen der Gallenblase und der Gallengänge sowie des Pankreasgangsystems. Hierbei wird - z. B. im Rahmen einer endoskopischen retrograden Cholangiographie (ERC) bzw. einer endoskopischen retrograden Cholangiopankreatikographie (ERCP) - ein Röntgenkontrastmittel unter Röntgenkontrolle in das zu untersuchende Gangsystem eingebracht.

Der Stand der Technik kennt flüssige Desinfektionsreiniger zur Reinigung und Desinfektion von medizinischen Instrumenten und flexiblen Endoskopen, welche neben Tensiden und Desinfektionswirkstoffen im wesentlichen organische Lösemittel, Säuren oder Laugen enthalten. Allerdings weisen die genannten Verbindungen einige Nachteile auf:

Lösemittel, Säuren und Laugen sind zwar von ihrer Wirksamkeit her zur Reinigung prinzipiell geeignet, haben aber den Nachteil, daß sie für das empfindliche Material zu aggressiv sind und medizinische Geräte oder Endoskope dementsprechend beschädigen können.

Die flüssigen Desinfektionsreiniger des Standes der Technik sind bei der Entfernung von Blut- und Sekretresten sowie bei der Ablösung von Fett und anderen kohlenwasserstoffhaltigen Verbindungen relativ gut wirksam. Keines der Desinfektionsmittel zeigt eine Kombination aus kurzer Einwirkzeit bei geringer Temperatur, niedriger Toxizität, geringer Sensibilisierung und Geruchsbildung sowie geringer Viskosität bei gleichzeitig optimaler Wirksamkeit gegenüber Bakterien wie insbesondere Mykobakterien und/oder Viren. Sie eignen sich auch nicht, um Reste von Röntgenkontrastmitteln vollständig zu entfernen.

Aufgabe der Erfindung war es daher, ein flüssiges, bevorzugt aldehydfreies, reinigendes und desinfizierendes System zu entwickeln, das die Nachteile des Standes der Technik nicht aufweist, welches also eine gute Reinigungsleistung und gleichzeitige oder nachfolgende Desinfektion bei zugleich hoher Materialverträglichkeit zeigt und bei geringer Einsatzkonzentration trotzdem eine gute Wirksamkeit gegenüber Grampositiven und Gramnegativen Bakterien, Pilzen, Viren und Mykobakterien aufweist.

Es wurde nun überraschend gefunden, daß alle diese Aufgaben gelöst werden durch ein reinigendes und desinfizierendes System, welches so auszugestalten ist, daß es
A) eine erste Zubereitung in Form eines flüssigen Reinigungsmittels für medizinische Instrumente, welches
   ■ gegebenenfalls mindestens ein nichtionisches Tensid und
   ■ mindestens eine Aminosäure und/oder ein Aminosäurenderivat enthält,
   und
B) eine zweite Zubereitung, welche in einem geeigneten Träger
   ■ mindestens einen mikrobiziden Wirkstoff, gewählt aus der Gruppe der Alkylamine
   ■ mindestens einen zweiten mikrobiziden Wirkstoff, gewählt aus der Gruppe der quatemären Ammoniumverbindungen
   enthält,
umfaßt.

Es war insbesondere überraschend, daß die erfindungsgemäßen reinigenden und desinfizierenden Systeme besser und schonender reinigen als die Zubereitungen des Standes der Technik und daß durch diese verbesserte Reinigung und gleichzeitige oder nachfolgende Desinfektion bei den überprüften flexiblen Endoskopen das mikrobiologische Problem des Standes der Technik wesentlich verringert werden konnte.

Gegenstand der Erfindung ist dementsprechend auch die Verwendung eines reinigenden und desinfizierenden Systems, welche so auszugestalten ist, daß es
A) eine erste Zubereitung in Form eines flüssigen Reinigungsmittels für medizinische Instrumente, welches
   ■ gegebenenfalls mindestens ein nichtionisches Tensid und
   ■ mindestens eine Aminosäure und/oder ein Aminosäurenderivat
   enthält,
   und
B) eine zweite Zubereitung, welche in einem geeigneten Träger
   ■ mindestens einen mikrobiziden Wirkstoff, gewählt aus der Gruppe der Alkylamine
   ■ mindestens einen zweiten mikrobiziden Wirkstoff, gewählt aus der Gruppe der quatemären Ammoniumverbindungen
   enthält,
umfaßt,
zur Reinigung und Desinfektion medizinischer Instrumente, flexibler Endoskope und medizinischer Oberflächen.

Die Dokumente US-A-5,403,505, WO-A-99/15012 und D4-WO-A-98/16605, in welchen Reinigungs-/Desinfektionszubereitungen beschrieben werden, konnten nicht den Weg zur vorliegenden Erfindung weisen.

Erfindungsgemäß vorteilhaft werden die quaternären Ammonium-Verbindungen bevorzugt gewählt aus der Gruppe Benzalkoniumchlorid, Didecyldimethylammoniumchtorid, Dioctyldimethylammoniumchlorid, Dimethyldecyloxethylammoniumpropionat, Cetrimoniumbromid, Cetylpyridiniumchlorid (Hexadecylpyridiniumchlorid).

Erfindungsgemäß vorteilhaft werden die Alkylamine bevorzugt gewählt aus der Gruppe der Fettaminderivate Laurylpropylendiamin und Dodecylbispropylentriamin, insbesondere bevorzugt ist das Dodecylbispropylentriamin.

Erfindungsgemäß vorteilhafte Aminosäuren sind z. B. die Glutaminsäure, welche sich durch die folgende Strukturformel auszeichnet: 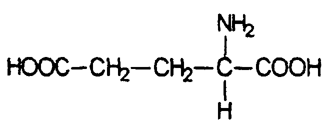 und/oder die Pyrrolidoncarbonsäure (Pyroglutaminsäure), welche sich durch die folgende Strukturformel auszeichnet

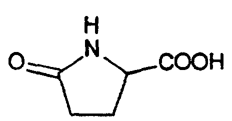

Vorteilhaft wird die Gesamtmenge an Aminosäuren (eine oder mehrere Verbindungen) aus dem Bereich von 0,1 bis 10,0 Gew.-%, vorzugsweise von 0,5 bis 2,0 Gew.-% gewählt, jeweils bezogen auf das Gesamtgewicht der Zubereitungen.

Erfindungsgemäß vorteilhaft werden zusätzlich nichtionische Tenside zugesetzt, insbesondere vorteilhaft gewählt aus der Gruppe der Alkylethoxylate, deren Alkylgruppe eine gesättigte oder ungesättigte, gerad- oder verzweigtkettige Alkylgruppe mit 8 bis 18, vorzugsweise 12 bis 14 Kohlenstoffatomen ist. Vorzugsweise enthalten sie bei verzweigtkettigen Alkylgruppen pro Molekül 2 bis 15, insbesondere 5 bis 9, speziell 7 Ethylenoxideinheiten enthalten und bei geradkettigen Alkylgruppen pro Molekül 2 bis 15, insbesondere 8 bis 14, speziell 12 Ethylenoxideinheiten. Ganz besonders bevorzugt sind Isotridecanolethoxylat und/oder Fettalkoholpolyglykolether.

Vorteilhaft wird die Gesamtmenge an nichtionischen Tensiden (eine oder mehrere Verbindungen) aus dem Bereich von 1,0 bis 20,0 Gew.-%, vorzugsweise von 5,0 bis 15,0 Gew.-% gewählt, jeweils bezogen auf das Gesamtgewicht der Zubereitungen.

Ein derart ausgestaltetes erfindungsgemäßes reinigendes und desinfizierendes System wird beispielsweise vorteilhaft in Form eines "Sets" verwirklicht, bei welchem die Zubereitungen A) und B) getrennt voneinander, in verschiedenen Behältern aufbewahrt werden.

Die Zubereitungen A) können in einem solchen Falle vorteilhaft so ausgestaltet werden, daß das oder die nichtionische Tenside gewählt werden aus der Gruppe der Alkylethoxylate, deren Alkylgruppe eine gesättigte oder ungesättigte, gerad- oder verzweigtkettige Alkylgruppe mit 8 bis 18, vorzugsweise 12 bis 14 Kohlenstoffatomen ist. Vorzugsweise enthalten sie bei verzweigtkettigen Alkylgruppen pro Molekül 2 bis 15, insbesondere 5 bis 9, speziell 7 Ethylenoxideinheiten enthalten und bei geradkettigen Alkylgruppen pro Molekül 2 bis 15, insbesondere 8 bis 14, speziell 12 Ethylenoxideinheiten. Ganz besonders bevorzugt sind Isotridecanolethoxylat und/oder Fettalkohol-polyglykolether. Vorteilhaft wird die Gesamtmenge an nichtionischen Tensiden (eine oder mehrere Verbindungen) aus dem Bereich von 1,0 bis 20,0 Gew.-%, vorzugsweise von 5,0 bis 15,0 Gew.-% gewählt, jeweils bezogen auf das Gesamtgewicht der Zubereitungen.

Vorteilhaft wird die Gesamtmenge an Aminosäuren (eine oder mehrere Verbindungen) aus dem Bereich von 0,1 bis 10,0 Gew.-%, vorzugsweise von 0,5 bis 2,0 Gew.-% gewählt, jeweils bezogen auf das Gesamtgewicht der Zubereitungen.

Die Zubereitungen A) können in Form von flüssigen Konzentraten vorliegen und beispielsweise in Dosierbeutel abgepackt werden, so daß eine Gebrauchslösung jeweils frisch angesetzt werden kann. Es ist insbesondere vorteilhaft, wenn der pH-Wert des Konzentrates zwischen 7 und 11 liegt. Die flüssigen Konzentrate werden vorzugsweise mit Wasser zu einer Gebrauchslösung verdünnt. Bevorzugt sind Gebrauchslösungen, die 0,5 bis 5 Gew.-% an erfindungsgemäßen reinigenden und desinfizierenden Systeme enthalten. Die Gebrauchslösung hat vorzugsweise einen pH-Wert zwischen 7 und 10, um eine optimale Schonung des empfindlichen Materials zu erreichen.

Vorteilhaft ist es, die Verdünnung so durchzuführen, daß der Gehalt der einzelnen Substanzen in der Gebrauchslösung wie folgt ist:
■ Alkylamine: zwischen 0,005 und 2,0 Gew.-%
■ Aminosäure: zwischen 0,005 und 0,5 Gew.-%
■ quaternäre Ammoniumverbindung zwischen 0,005 und 2,0 Gew.-%
■ nichtionische Tenside: zwischen 0,01 und 1,0 Gew.-%.

Die Zubereitungen A) können ferner vorteilhaft zusätzlich übliche Korrosionsinhibitoren, wie beispielsweise Benzotriazol, enthalten, insbesondere falls sie bei ihrer Anwendung mit metallischen Oberflächen, insbesondere mit nicht veredelten Stählen oder Werkstoffen aus Buntmetallegierungen, in Berührung kommen können. Es wird bevorzugt, den Gehalt an einem oder mehreren Korrosionsinhibitoren zwischen 0,1 und 5 Gew.-%, bezogen auf das Gesamtgewicht der Formulierung, zu wählen.

Die Zubereitungen A) können darüber hinaus vorteilhaft zusätzlich Komplexbildner und/oder pH-Regulatoren enthalten, um den negativen Einfluß schwankender Wasserhärte zu reduzieren bzw. um zu gewährleisten, daß der pH-Wert des reinigenden und desinfizierenden Systems in einem Bereich liegt, der das empfindliche Material nicht schädigt.

Zusätzlich zu den vorstehend genannten Komponenten können die Zubereitungen A) für derartige Zubereitungen übliche Konservierungsstoffe, Farbstoffe, Duftstoffe und/oder andere übliche Hilfsstoffe enthalten.

Die jeweils einzusetzenden Mengen an derartigen Trägerstoffen und Parfüm können in Abhängigkeit von der Art des jeweiligen Produktes vom Fachmann durch einfaches Ausprobieren leicht ermittelt werden.

Die Zubereitungen B) enthalten ein oder mehrere mikrobizide Wirkstoffe in einem geeigneten Träger. Sie können in Form von festen Konzentraten, z. B. in (Brause-) Tabletten-, Pulver-, Pellet- oder Granulatform, vorliegen und beispielsweise in Dosierbeutel abgepackt werden, so daß eine Gebrauchslösung jeweils frisch angesetzt werden kann. Sie können aber auch in Form von Lösungen oder Suspensionen in nicht-wäßrigen Lösungsmitteln vorliegen.

Bevorzugt sind Zubereitungen B), welche auch Tenside enthalten, wie sie üblicherweise Verwendung finden, beispielsweise nichtionische Tenside auf Basis von Ethylenoxid und/oder Propylenoxid und/oder Butylenoxid.

Ferner können die Zubereitungen B) vorteilhaft zusätzlich übliche Korrosionsinhibitoren, wie beispielsweise Benzotriazol enthalten, insbesondere falls sie bei ihrer Anwendung mit metallischen Oberflächen, insbesondere mit nicht veredelten Stählen oder Werkstoffen aus Buntmetallegierungen, in Berührung kommen können.

Zusätzlich zu den vorstehend genannten Komponenten können die Zubereitungen B) für derartige Zubereitungen übliche Farbstoffe, Duftstoffe und/oder andere übliche Hilfsstoffe, wie beispielsweise Dickungs-, Granulierungs-, Tablettierungs- oder Dispergierhilfsmittel, enthalten. Diese tragen in der Mehrzahl der Fälle nicht zur desinfizierenden, konservierenden oder antiseptischen Wirkung bei, sondern dienen der Handhabbar- bzw. Lagerbarkeit sowie ästhetischen Zwecken. Es ist jedoch auch möglich, solche Komponenten zu verwenden, die eine (konservierende, pflegende) Wirkung entfalten und dabei gleichzeitig für eine bestimmte Farbe und/oder einen angenehmen Duft sorgen.

Die jeweils einzusetzenden Mengen an derartigen Trägerstoffen und Parfüm können in Abhängigkeit von der Art des jeweiligen Produktes vom Fachmann durch einfaches Ausprobieren leicht ermittelt werden.

Die folgenden Beispiele sind keine Beispiele gemäß der Lösung der Aufgabe. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele:

### Beispiel 1:

| | |
|---|---|
| 9,5 | Didecyldimethylammoniumchlorid |
| 14 | Dodecylbispropylentriamin |
| 1 % | Pyrrolidoncarbonsäure |
| 3 % | Isotridecanolethoxylat |
| 4 % | Fettalkoholpolyglykolether |
| 1,5 % | Korrosionsschutz |
| 5 % | Glykol |
| 7 % | pH-Regulator |
| 1 % | Komplexbildner |
| Rest | Wasser |

Es wird ein pH-Wert von 9 eingestellt

### Beispiel 2:

| | |
|---|---|
| 13 % | Didecyldimethylammoniumchlorid |
| 9 % | Dodecylbispropylentriamin |
| 1 % | Pyrrolidoncarbonsäure |
| 3 % | Isotridecanolethoxylat |
| 4 % | Fettalkoholpolyglykolether |
| 1,5 % | Korrosionsschutz |
| 5 % | Glykol |
| 6,5 % | pH-Regulator |
| 1 % | Komplexbildner |
| Rest | Wasser |

Es wird ein pH-Wert von 9 eingestellt.

### Beispiel 3:

| | |
|---|---|
| 13 % | Didecyldimethylammoniumchlorid |
| 9 % | Dodecylbispropylentriamin |
| 5% | Pyrrolidoncarbonsäure |
| 3 % | Isotridecanolethoxylat |
| 4 % | Fettalkoholpolyglykolether |
| 1,5 % | Korrosionsschutz |
| 5 % | Glykol |
| 6,5 % | pH-Regulator |
| 1 % | Komplexbildner |
| Rest | Wasser |

Es wird ein pH-Wert von 9 eingestellt.

### Beispiel 4:

| | |
|---|---|
| 13 % | Didecyldimethylammoniumchlorid |
| 9 % | Dodecylpropylendiamin |
| 3 % | Isotridecanolethoxylat |
| 4 % | Fettalkoholpolyglykolether |
| 1,5 % | Korrosionsschutz |
| 5 % | Glykol |
| 7 % | pH-Regulator |
| 1 % | Komplexbildner |
| Rest | Wasser |

Es wird ein pH-Wert von 9 eingestellt

## Patentansprüche

1. Reinigendes und desinfizierendes System, welches so auszugestalten ist, daß es
A) eine erste Zubereitung in Form eines flüssigen Reinigungsmittels für medizinische Instrumente, welches
■ gegebenenfalls mindestens ein nichtionisches Tensid und
■ mindestens eine Aminosäure und/oder ein Aminosäurenderivat enthält,
und
B) eine zweite Zubereitung, welche in einem geeigneten Träger
■ mindestens einen mikrobiziden Wirkstoff, gewählt aus der Gruppe der Alkylamine
■ mindestens einen zweiten mikrobiziden Wirkstoff, gewählt aus der Gruppe der quatemären Ammoniumverbindungen
enthält,
umfaßt.

2. Reinigendes und desinfizierendes System nach Anspruch 1, **dadurch gekennzeichnet, daß** das oder die Alkylamine gewählt werden aus der Gruppe Dodecylbispropylentriamin, Laurylpropylendiamin.

3. Reinigendes und desinfizierendes System nach Anspruch 1, **dadurch gekennzeichnet, daß** die quaternären Ammoniumverbindungen gewählt werden aus der Gruppe Benzalkoniumchlorid, Didecyldimethylammoniumchlorid, Dioctyldimethylammoniumchlorid, Dimethyldecyloxethyl-ammoniumpropionat, Cetrimoniumbromid, Cetylpyridiniumchlorid (Hexadecylpyridiniumchlorid).

4. Reinigendes und desinfizierendes System nach einem der vorhergehende Ansprüche, **dadurch gekennzeichnet, daß** es als Aminosäure Pyrrolidoncarbonsäure und/oder Glutaminsäure enthält.

5. Reinigendes und desinfizierendes System nach einem der vorhergehende Ansprüche, **dadurch gekennzeichnet, daß** das oder die nichtionischen Tenside gewählt werden aus der Gruppe der Alkylethoxylate und/oder Alkylpropoxylate, deren Alkylgruppe eine gesättigte oder ungesättigte, gerad- oder verzweigtkettige Alkylgruppe mit 8 bis 18, vorzugsweise 12 bis 14 Kohlenstoffatomen ist, bevorzugt Isotridecanolethoxylat und/oder Fettalkoholpolyglykolether.

6. Reinigendes und desinfizierendes System nach einem der vorhergehende Ansprüche, **dadurch gekennzeichnet, daß** es einen odere mehrere Wirkstoffe aus der Gruppe der quaternären Ammoniumverbindungen und der Gruppe der Alkylamine enthält in einer Menge von 1,0 bis 40,0 Gew.-%, vorzugsweise von 5,0 bis 25,0 Gew.-%, jeweils bezogen auf das Gesamtgewicht der Zubereitungen, enthält.

7. Reinigendes und desinfizierendes System nach einem der der vorhergehende Ansprüche, **dadurch gekennzeichnet, daß** es ein oder mehrere nichtionische Tenside in einer Menge von 1,0 bis 20,0 Gew.-%, vorzugsweise von 5,0 bis 15,0 Gew.-%, jeweils bezogen auf das Gesamtgewicht der Zubereitungen, enthält.

8. Reinigendes und desinfizierendes System nach einem der vorhergehende Ansprüche, **dadurch gekennzeichnet, daß** es eine oder mehrere Aminosäuren in einer Menge von 0,1 bis 10,0 Gew.-%, vorzugsweise von 0,5 bis 2,0 Gew.-%, jeweils bezogen auf das Gesamtgewicht der Zubereitungen, enthält.

9. Reinigendes und desinfizierendes System nach einem der vorhergehende Ansprüche, **dadurch gekennzeichnet, daß** es ausserdem übliche Zusatzstoffe wie Lösungsmittel, Lösungsvermittler, Tenside, Korrosionsinhibitoren, Komplexbildner, pH-Regulatoren, Schaumregulatoren, Parfüm und/oder Farbstoffe enthält.

10. Reinigendes und desinfizierendes System nach einem der vorhergehende Ansprüche, **dadurch gekennzeichnet, daß** es einen pH-Wert zwischen 7 und 11 hat.

11. Reinigendes und desinfizierendes System nach mindestens einem der vorhergehenden Ansprüche, **dadurch gekennzeichnet, daß** es in Form eines Konzentrats vorliegt, welches vor der Verwendung mit Wasser zu einer Gebrauchslösung verdünnt wird.

12. Verwendung eines reinigenden und desinfizierenden Systems, welche so auszugestalten ist, daß es
A) eine erste Zubereitung in Form eines flüssigen Reinigungsmittels für medizinische Instrumente, welches
■ gegebenenfalls mindestens ein nichtionisches Tensid und
■ mindestens eine Aminosäure und/oder ein Aminosäurenderivat
enthält,
und
B) eine zweite Zubereitung, welche in einem geeigneten Träger
■ mindestens einen mikrobiziden Wirkstoff, gewählt aus der Gruppe der Alkylamine
■ mindestens einen zweiten mikrobiziden Wirkstoff, gewählt aus der Gruppe der quatemären Ammoniumverbindungen
enthält,
umfaßt,
zur Reinigung und Desinfektion medizinischer Instrumente, flexibler Endoskope und medizinischer Oberflächen.

## Claims

1. Cleaning and disinfecting system, which is to be equipped such that it comprises
A) a first preparation in the form of a liquid cleaning agent for medical instruments, which
■ optionally contains at least one non-ionic surfactant and
■ at least one amino acid and/or one amino acid derivative,
and
B) a second preparation, which in a suitable carrier
■ contains at least one microbicidal active compound, chosen from the group consisting of the alkylamines
■ at least one second microbicidal active compound, chosen from the group consisting of the quaternary ammonium compounds.

2. Cleaning and disinfecting system according to Claim 1, **characterized in that** the alkylamine(s) are chosen from the group consisting of dodecylbispropylenetriamine, laurylpropylenediamine.

3. Cleaning and disinfecting system according to Claim 1, **characterized in that** the quaternary ammonium compounds are chosen from the group consisting of benzalkonium chloride, didecyldimethylammonium chloride, dioctyldimethylammonium chloride, dimethyldecyloxethylammonium propionate, cetrimonium bromide, cetylpyridinium chloride (hexadecylpyridinium chloride).

4. Cleaning and disinfecting system according to one of the preceding claims, **characterized in that** the amino acid contained is pyrrolidonecarboxylic acid and/or glutamic acid.

5. Cleaning and disinfecting system according to one of the preceding claims, **characterized in that** the non-ionic surfactants are chosen from the group consisting of the alkyl ethoxylates and/or alkyl propoxylates, whose alkyl group is a saturated or unsaturated, straight- or branched-chain alkyl group having 8 to 18, preferably 12 to 14, carbon atoms, preferably isotridecanol ethoxylate and/or fatty alcohol polyglycol ethers.

6. Cleaning and disinfecting system according to one of the preceding claims, **characterized in that** it contains one or more active compounds from the group consisting of the quaternary ammonium compounds and the group consisting of the alkylamines in an amount from 1.0 to 40.0% by weight, preferably from 5.0 to 25.0% by weight, in each case based on the total weight of the preparations.

7. Cleaning and disinfecting system according to one of the preceding claims, **characterized in that** it contains one or more non-ionic surfactants in an amount from 1.0 to 20.0% by weight, preferably from 5.0 to 15.0% by weight, in each case based on the total weight of the preparations.

8. Cleaning and disinfecting system according to one of the preceding claims, **characterized in that** it contains one or more amino acids in an amount from 0.1 to 10.0% by weight, preferably from 0.5 to 2.0% by weight, in each case based on the total weight of the preparations.

9. Cleaning and disinfecting system according to one of the preceding claims, **characterized in that** it moreover contains customary additives such as solvents, solubilizers, surfactants, corrosion inhibitors, complexing agents, pH regulators, foam regulators, perfume and/or colourants.

10. Cleaning and disinfecting system according to one of the preceding claims, **characterized in that** it has a pH of between 7 and 11.

11. Cleaning and disinfecting system according to at least one of the preceding claims, **characterized in that** it is present in the form of a concentrate which is diluted before use with water to give a use solution.

12. Use of a cleaning and disinfecting system which is to be equipped such that it comprises
A) a first preparation in the form of a liquid cleaning agent for medical instruments, which
■ optionally contains at least one non-ionic surfactant and
■ at least one amino acid and/or one amino acid derivative,
and
B) a second preparation, which in a suitable carrier
■ contains at least one microbicidal active compound, chosen from the group consisting of the alkylamines
■ at least one second microbicidal active compound, chosen from the group consisting of the quaternary ammonium compounds,
for the cleaning and disinfection of medical instruments, flexible endoscopes and medical surfaces.

## Revendications

1. Système détergent et désinfectant qui est constitué de telle manière qu'il comprend:
A) une première préparation sous forme d'un produit de nettoyage liquide pour instruments médicaux, qui contient
■ éventuellement au moins un tensioactif non ionique et
■ au moins un aminoacide et/ou un dérivé d'amino-acide, et
B) une seconde préparation qui contient, dans un véhicule convenable
■ au moins une substance active microbicide, choisie dans le groupe des alkylamines
■ au moins une seconde substance active microbicide, choisie dans le groupe des composés d'ammonium quaternaire.

2. Système détergent et désinfectant selon la revendication 1, **caractérisé en ce que** la ou les alkylamines sont choisies dans le groupe constitué par la dodécyl-bis-propylènetriamine et la laurylpropylènediamine.

3. Système détergent et désinfectant selon la revendication 1, **caractérisé en ce que** les composés d'ammonium quaternaire sont choisis dans le groupe constitué par le chlorure de benzalkonium, le chlorure de didécyldiméthylammonium, le chlorure de dioctyldiméthylammonium, le propionate de diméthyldécyloxyéthylammonium, le bromure de cétrimonium, le chlorure de cétylpyridinium (chlorure d'hexadécylpyridinium).

4. Système détergent et désinfectant selon l'une quelconque des revendications précédentes, **caractérisé en ce qu'**il contient en tant qu'aminoacide de l'acide pyrrolidonecarboxylique et/ou de l'acide glutamique.

5. Système détergent et désinfectant selon l'une quelconque des revendications précédentes, **caractérisé en ce que** le ou les agents tensioactifs sont choisis dans le groupe des alkyléthoxylates et/ou des alkylpropoxylates, dont le groupe alkyle est un groupe alkyle saturé ou insaturé, à chaîne droite ou ramifiée, ayant de 8 à 18, de préférence de 12 à 14 atomes de carbone, de préférence l'éthoxylate d'isotridécanol et/ou le polyglycoléther d'alcool gras.

6. Système détergent et désinfectant selon l'une quelconque des revendications précédentes, **caractérisé en ce qu'**il contient une ou plusieurs substances actives choisies dans le groupe des composés d'ammonium quaternaire et dans le groupe des alkylamines, en une quantité de 1,0 à 40,0 % en poids, de préférence de 5,0 à 25,0 % en poids, chaque fois par rapport au poids total des préparations.

7. Système détergent et désinfectant selon l'une quelconque des revendications précédentes, **caractérisé en ce qu'**il contient un ou plusieurs tensioactifs non ioniques en une quantité de 1,0 à 20,0 % en poids, de préférence de 5,0 à 15,0 % en poids, chaque fois par rapport au poids total des préparations.

8. Système détergent et désinfectant selon l'une quelconque des revendications précédentes, **caractérisé en ce qu'**il contient un ou plusieurs aminoacides en une quantité de 0,1 à 10,0 % en poids, de préférence de 0,5 à 2,0 % en poids, chaque fois par rapport au poids total des préparations.

9. Système détergent et désinfectant selon l'une quelconque des revendications précédentes, **caractérisé en ce qu'**il contient en outre des additifs usuels tels que des solvants, des tiers-solvants, des tensioactifs, des inhibiteurs de corrosion, des complexants, des régulateurs de pH, des régulateurs de mousse, un parfum et/ou des colorants.

10. Système détergent et désinfectant selon l'une quelconque des revendications précédentes, **caractérisé en ce qu'**il présente un pH compris entre 7 et 11.

11. Système détergent et désinfectant selon au moins l'une des revendications précédentes, **caractérisé en ce qu'**il se trouve sous forme d'un concentré qui, avant l'emploi, est dilué avec de l'eau pour donner une solution prête à l'emploi.

12. Utilisation d'un système détergent et désinfectant qui est constitué de telle manière qu'il comprend:
A) une première préparation sous forme d'un produit de nettoyage liquide pour instruments médicaux, qui contient
■ éventuellement au moins un tensioactif non ionique et
■ au moins un aminoacide et/ou un dérivé d'amino-acide, et
B) une seconde préparation qui contient, dans un véhicule convenable
■ au moins une substance active microbicide, choisie dans le groupe des alkylamines
■ au moins une seconde substance active microbicide, choisie dans le groupe des composés d'ammonium quaternaire,
pour le nettoyage et la désinfection d'instruments médicaux, d'endoscopes flexibles et de surfaces médicales
